(19) Europäisches Patentamt

European Patent Office

Office européen des brevets

(11) EP 1 094 415 A2

(12) EUROPÄISCHE PATENTANMELDUNG

(43) Veröffentlichungstag:
25.04.2001 Patentblatt 2001/17

(51) Int. Cl.⁷: G06F 19/00

(21) Anmeldenummer: 00122333.8

(22) Anmeldetag: 23.10.2000

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 21.10.1999 US 422303
29.02.2000 DE 10009479

(71) Anmelder: 4SC GmbH
82152 Planegg-Martinsried (DE)

(72) Erfinder:
• Vitt, Daniel
82152 Planegg-Martinsried (DE)

• Busemann, Stefan
81375 München (DE)
• Dauer, Ulrich
84424 Isen (DE)

(74) Vertreter:
Bösl, Raphael, Dr. rer. nat., Dipl.-Chem. et al
Patent- und Rechtsanwälte
Bardehle . Pagenberg . Dost . Altenburg .
Geissler . Isenbruck
Galileiplatz 1
81679 München (DE)

(54) **Verfahren zur Identifizierung von Kandidatenmolekülen**

(57) Die vorliegende Erfindung betrifft ein Verfahren zur Identifizierung von Kandidatenmolekülen mit erwarteter biologischer Aktivität, bei dem man: einen Satz aus verschiedenen Molekülen erzeugt; jedem der Moleküle des Satzes einen Deskriptor zuordnet, der eine vorbestimmte Zahl von molekularen Eigenschaften repräsentiert; den Molekülsatz auf Punkten eines zweidimensionalen Gitters hinsichtlich einer vorbestimmten Similaritätsbeziehung der jeweils zugeordneten Deskriptoren so kartiert, daß der Gitterabstand zwischen Gitterpunkten zweier Moleküle ein Maß für die Similarität der beiden Moleküldeskriptoren darstellt; über dem Molekülgitter eine dreidimensionale Oberfläche erstellt, die die Verteilung der biologischen Aktivität der Moleküle auf dem Gitter gemäß einem vorbestimmten Qualitätskriterium approximativ darstellt; und aus der dreidimensionalen Oberfläche Kandidatenmoleküle auswählt, die ein vorbestimmtes Kriterium hinsichtlich ihrer biologischen Aktivität erfüllen.

Fig. 6

EP 1 094 415 A2

**Beschreibung**

Die vorliegende Erfindung betrifft ein Verfahren zur Identifizierung von Kandidatenmolekülen mit erwarteter biologischer Aktivität.

In der pharmazeutischen Wirkstoff-Forschung stellt die Evaluierung der Rezeptor- oder Targeteignung von Molekülen eine wichtige Aufgabe dar. Mit dem bei Wirkstoff-Findung im Lauf der letzten Jahre zunehmenden Einsatz von Automatisierungstechniken haben sich Verfahren wie das Hochdurchsatz-Screening (HTS, High Throughput Screening) und die Hochdurchsatz-Synthese in der pharmazeutischen Forschung zum Industriestandard entwickelt. Heutzutage kann man über 20.000 Moleküle pro Tag auf ihre biologischen Aktivitäten bei bestimmten Zielkrankheiten prüfen. Auch auf dem Gebiet der chemischen Synthese können mit Hilfe der kombinatorischen Chemie in Kombination mit Automatisierungsverfahren hunderte von Molekülen pro Tag physisch zur Verfügung gestellt werden. Da ausgehend vom derzeitigen chemischen Wissen theoretisch mehr als $10^{100}$ Moleküle synthetisiert und geprüft werden könnten und mehrere hunderttausend Moleküle im Handel erhältlich sind, wurden computergestütze Verfahren zur Auswahl von Untergruppen von Molekülen, die nun tatsächlich geprüft werden sollen, auf der Basis ihres prognostizierten biologischen Aktivitätspotentials für bestimmte Zielkrankheiten entwickelt.

Zwei Kategorien von computergestützten Verfahren dienen zur Auffindung (Auswahl und/oder Prioritisierung) von Molekülen aus Datensätzen von theoretisch verfügbaren Molekülen für die Prüfung der biologischen Aktivität. Die erste Kategorie umfaßt auf Diversität oder Similarität beruhende Auffindungsverfahren, wohingegen die zweite Kategorie auf der Struktur basierende Auffindungsverfahren umfaßt. Zur zweiten Kategorie gehören Datenbanksuchtechniken sowie (Q)SAR- und Docking-Verfahren.

Nur die (Q)SAR- und Docking-Verfahren berücksichtigen implizit Informationen bezüglich spezifischer Targets, entweder übliche Strukturmuster einer Reihe von aktiven Molekülen ((Q)SAR) oder die dreidimensionale Struktur eines Targetproteins (Docking), und liefern daher die genauesten Ergebnisse. In der Praxis werden auf (Q)SAR oder Docking beruhende Verfahren auf kleinere Datensätze (bis zu 50.000 Sätze) angewandt, da sie eine verhältnismäßig hohe Rechenleistung erfordern. Wenngleich die Geschwindigkeit mit Parallelrechentechniken gesteigert werden kann, sind Datensätze aus mehr als $10^6$ Molekülen hinsichtlich ihrer biologischen Aktivität immer noch nicht in einem vertretbaren zeitlichen Rahmen prognostizierbar.

Der Begriff biologische Aktivität umfaßt im folgenden insbesondere pharmazeutische sowie agrochemische Aktivität bezüglich eines bestimmten Rezeptors oder Targets.

Die Suche nach Kandidatenmolekülen umfaßt auch die Suche nach Leitverbindungen.

Der vorliegenden Erfindung liegt daher die Aufgabe zugrunde, ein auf Molekülbibliotheken mit großen Datenmengen anwendbares und in vertretbarer Zeit Ergebnisse lieferndes Verfahren und System zur Auffindung von Kandidatenmolekülen mit erwarteter biologischer Aktivität bereitzustellen.

Diese Aufgabe wird mit dem Verfahren und dem System gemäß den unabhängigen Ansprüchen gelöst. Vorteilhafte Ausführungsformen sind in den Unteransprüchen definiert.

Erfindungsgemäß geht man bei dem Verfahren zur Identifizierung von Kandidatenmolekülen mit erwarteter biologischer Aktivität so vor, daß man:

a) einen Satz aus verschiedenen Molekülen erzeugt;

b) jedem der Moleküle des Satzes einen Deskriptor zuordnet, der eine vorbestimmte Zahl von molekularen Eigenschaften repräsentiert;

c) den Molekülsatz auf Punkten eines zweidimensionalen Gitters hinsichtlich einer vorbestimmten Similaritätsbeziehung der jeweils zugeordneten Deskriptoren so kartiert, daß der Gitterabstand zwischen Gitterpunkten zweier Moleküle ein Maß für die Similarität der beiden Moleküldeskriptoren darstellt;

d) über dem Molekülgitter eine dreidimensionale Oberfläche erstellt, die die Verteilung der biologischen Aktivität der Moleküle auf dem Gitter gemäß einem vorbestimmten Qualitätskriterium approximativ darstellt; und

e) aus der dreidimensionalen Oberfläche Kandidatenmoleküle auswählt, die ein vorbestimmtes Kriterium hinsichtlich ihrer biologischen Aktivität erfüllen.

Erfindungsgemäß kann man in Schritt d) die dreidimensionale Oberfläche dadurch erstellen, daß man:

da) als Anfangsbereich für die Approximation das gesamte zweidimensionale Gitter nimmt;

db) auf vorbestimmten Gitterpunkten dieses Bereichs Moleküle auswählt und deren jeweilige Werte für die biologische Aktivität berechnet;

dc) die Oberfläche unter Verwendung der vorher bestimmten Werte für die biologische Aktivität der Moleküle auf den vorbestimmten Gitterpunkten über diesen Bereich approximiert und

dd) bestimmt, ob die approximierte Oberfläche ein vorbestimmtes Qualitätskriterium erfüllt; wenn dies der Fall ist, geht man zu Schritt e); wenn dies nicht der Fall ist, verfeinert man die Approximation der Oberfläche durch Auswahl von Molekülen auf weiteren Gitterpunkten, Berechnung ihrer jeweiligen Werte für die biologische Aktivität und Wiederholung von Schritt dc) und diesem Schritt dd).

Die Erstellung der dreidimensionalen Oberfläche in Schritt d) erfolgt vorzugsweise durch eine Approximation nach der Methode der Delauney-Triangulierung.

Das erfindungsgemäße Verfahren besteht somit aus der Durchführung von zwei Hauptschritten. Im ersten Schritt werden die Moleküle der Similarität ihrer Deskriptoren entsprechend sortiert und auf einem 2-D-Gitter kartiert. Im zweiten Schritt wird die biologische Aktivität der kartierten Moleküle durch Modellierung der Verteilung als Oberfläche über die Molekülkarte approximiert. Aus der Oberfläche kann man geeignete Kandidatenmoleküle für die weitere Evaluierung bestimmen. Erfindungsgemäß muß nur eine kleine Molekülmenge im Datensatz tatsächlich berechnet werden. Dadurch ergibt sich eine beträchtliche Steigerung der Leistungsfähigkeit. Die rekursive Vorgehensweise ermöglicht die Untersuchung der Datenbank auf Basis von speziell anpaßbaren Qualitätskriterien. Fehler- und Qualitätskriterien für die Analyse können genau auf ein gegebenes Problem abgestellt werden. Parallel dazu können leicht Docking-Simulationen von Molekülsammlungen durchgeführt werden, was zu einer weiteren Leistungssteigerung führt.

Somit überträgt das erfindungsgemäße Verfahren prognostizierte/gemessene biologische Aktivität in topographische Informationen auf einer dreidimensionalen Oberfläche, die unter Verwendung von Approximationsalgorithmen iterativ analysiert wird. Eine gründliche Analyse erfolgt nur in denjenigen Bereiche der Oberfläche, die Bereichen hoher biologischer Aktivität darstellen, wohingegen die Bereiche mit niedrigen Bindungsenergien für eine gegebene Proteinbindungsstelle nur durch wenige Datenpunkte approximiert werden. Wie Beispiele gezeigt haben, kann man somit durch explizite Berechnung/Vermessung von nur 4-6% der Moleküle im Datensatz aktive Moleküle aus Datensätzen identifizieren.

Mit dem erfindungsgemäßen Verfahren kann man Wirkstoff-Leitkandidaten identifizieren, ohne große Molekülsätze physisch zur Verfügung stellen und prüfen zu müssen.

Die gewählten Kandidatenmoleküle sind für die chemische Synthese geeignet.

Vorzugsweise handelt es sich bei den von den Deskriptoren dargestellten molekularen Eigenschaften um mindestens zwei der folgenden Eigenschaften:

- Molekulargewicht,
- Zahl der drehbaren Bindungen,
- Zahl der hydrophoben Gruppen,
- Zahl der hydrophilen Gruppen,
- Zahl der sauren Gruppen,
- Zahl der basischen Gruppen,
- Zahl der neutralen Gruppen,
- Zahl der Zwittergruppen,
- Zahl der Schweratome,
- Zahl der H-Bindungs-Donatoren,
- Zahl der H-Bindungs-Akzeptoren,
- Zahl der 1,2-Dipole,
- Zahl der 1,3-Dipole,
- Zahl der 1,4-Dipole.

Die Molekülkartierung erfolgt vorzugsweise mit selbstorganisierenden Karten in einem neuronalen Netz oder mit statistischen Methoden wie linearer Vektorquantisierung.

Einen weiteren Gegenstand der Erfindung bildet ein Computersystem mit Mitteln zur Durchführung des Identifizierungsverfahrens, Mitteln zur Eingabe von Systembefehlen und Mitteln zur Ausgabe des Ergebnisses der Durchführung des Verfahrens.

Die Erfindung und Beispiele dafür werden nun anhand der beigefügten Zeichnungen naher erläutert. Es zeigen:

Fig. 1 den hier verwendeten Deskriptortyp,
Fig. 2 die sich aus einem Molekülsortierschritt ergebende Verteilung,
Fig. 3 den Entclusterungsschritt,
Fig. 4 die sich aus der Entclusterung ergebende Verteilung,
Fig. 5 vier Zwischenschritte der Approximation einer Oberfläche durch Delauney-Triangulierung,
Fig. 6 die abschließende Approximation der Oberfläche durch Delauney-Triangulierung,
Fig. 7 einen sortierten Datensatz auf der Karte für ein Beispiel eines Datensatzes von 40.000 Molekülen,

Fig. 8 die Menge aktiver Moleküle für ein erstes Target gemäß dem Beispiel von Fig. 7,

Fig. 9 die Menge aktiver Moleküle für ein zweites Target gemäß dem Beispiel von Fig. 7,

Fig. 10 und 11 den Prozentsatz aktiver Moleküle bezogen auf die Gesamtmenge aktiver Moleküle, die in dem Datensatz enthalten sind, für die Ergebnisse gemäß Fig. 8 bzw. Fig. 9.

Das erfindungsgemäße Verfahren wird anhand des Beispiels der Auffindung von Inhibitoren von Dihydrofolatreductase (PDB-Code: 4 dfr) erläutert.

Erfindungsgemäß wird vor der Evaluierung von speziellen Molekülen die sogenannte virtuelle Bibliothek erzeugt, die alle möglichen Moleküle enthält. Das bedeutet, daß die virtuelle Molekülbibliothek Moleküle, die mit vertretbaren Kosten käuflich erworben oder hergestellt werden können und handelsübliche Moleküle oder mittels kombinatorischer Synthese herstellbare Moleküle enthält. Nicht enthalten sind die Moleküle, die von vornherein für die Wirkstoffsynthese ungeeignet sind, insbesondere Moleküle mit toxischen Gruppen, einem Molekulargewicht von mehr als 500 u oder mehr als 5 Donatoren oder einem log-P-Wert von mehr als 5. Die Bibliothek wird in einer Computerdatenbank gespeichert.

In diesem Beispiel enthält die Datenbank 2000 Moleküle. Jedes der Moleküle wird durch 2-D-Strukturdaten in maschinenlesbarer Form dargestellt.

Bei der Speicherung der Moleküle in der Bibliothek wird jedem Molekül in der Bibliothek ein Deskriptor zugeordnet, der mit der biologischen Aktivität des jeweiligen Moleküls in Relation steht. Bei dem Deskriptor handelt es sich um einen Vektor mehrerer skalarer molekularer Eigenschaften. Dieser Vektor umfaßt die folgenden Werte:

- Molekulargewicht,
- Zahl der drehbaren Bindungen,
- Zahl der hydrophoben Gruppen,
- Zahl der Schweratome,
- Zahl der H-Bindungs-Donatoren,
- Zahl der H-Bindungs-Akzeptoren.

Zur Durchführung einer Vorauswahl von Molekülen kann man Werte verwenden, die wirtschaftliche oder technische Aspekte abdecken, wie z.B. Verfügbarkeit und Herstellungskosten von Molekülen.

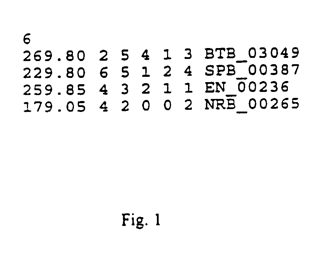

Fig. 1 zeigt vier Datenströme (die vier Moleküle beschreiben) des hier verwendeten Deskriptors. Die ersten Zeilen geben die Dimension des Deskriptors an, die zweiten bis fünften Zeilen geben die Moleküle an, und die letzte Spalte enthält die Identifizierung der entsprechenden Moleküle.

Die Deskriptoren sind auf die weitere Verarbeitung der Molekülbibliothek zwecks Auffindung der Molekülkandidaten für die Wirkstoffsynthese ausgelegt. Um eine weitere Verarbeitung zu ermöglichen, haben die für die Moleküle der Datenbank gewählten Deskriptoren alle die gleiche Dimension.

Im nächsten Schritt werden die Moleküle dieser Bibliothek ihrer Similarität hinsichtlich potentieller Ligand-Rezeptor-Aktivität entsprechend sortiert. Dies geschieht durch Kartieren der Moleküle auf einem zweidimensionalen Gitter, wobei die Similarität der Moleküle miteinander durch die Abstände ihrer Positionen auf dem Gitter dargestellt wird. Die Bestimmung der Similarität erfolgt durch Evaluierung der Deskriptoren der Moleküle. Die Sortierung basiert auf einem neuronalen Netz vom Typ der selbstorganisierenden Karte nach Kohonen. Selbstorganisierende Karten eignen sich gut, da sich herausgestellt hat, daß sie selbst bei Auftreten von linearen Abhängigkeiten in den Daten höherdimensionale Probleme auf niedrigere Dimensionen projizieren können.

Das Sortieren wird folgendermaßen durchgeführt: Zunächst werden alle Moleküle willkürlich auf das Gitter gesetzt. Das Gitter muß groß genug sein, um alle Moleküle der Datenbank aufzunehmen. Während der Lernphasen des neuronalen Netzes werden die Moleküle über das Gitter verschoben, um sie bezüglich ihrer Similarität zu ordnen. Hierbei werden drei Lernphasen angewandt. In der ersten Lernphase ist die Verschiebung auf einen Gitterradius von etwa 1/10 der Höchstzahl der Gitterpunkte in einer Richtung beschränkt, wobei die Konvergenz 0,3 beträgt. In der zweiten Lernphase beläuft sich der Radius auf einen Gitterpunkt und die Konvergenz auf 0,2; in der dritten Lernphase beläuft sich der Radius auf 0,5 und die Konvergenz auf 0,2. Der Gitterradius und die Konvergenz können selbstverständlich auf das konkrete Problem abgestellt werden.

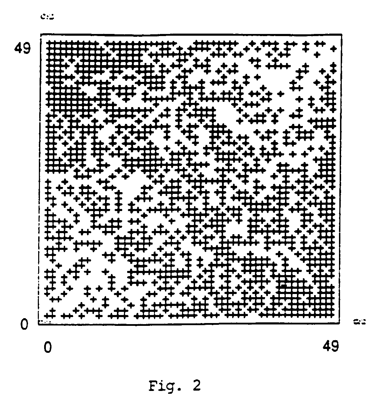

Als Ergebnis der Sortierung ergibt sich eine Molekülverteilung über das Gitter, bei der ein Gitterpunkt von mehreren Molekülen besetzt sein kann, wohingegen andere Gitterpunkte frei bleiben können. Fig. 2 zeigt die nach drei Lernphasen erhaltene Karte.

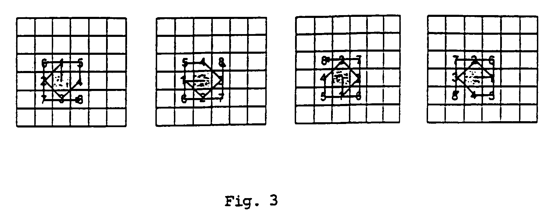

Wenngleich eine derartige unregelmäßige Besetzung der Molekülkarte die weitere Verarbeitung der Molekülkarte eigentlich nicht behindert, wird die Molekülkarte entclustert, d.h. Moleküle, die mit anderen zusammen auf dem gleichen Gitterpunkt liegen, werden auf freie Gitterpunkte in der näheren Umgebung gesetzt. Hierzu führt man nach folgendem Schema eine Suche nach einem freien Gitterpunkt durch (siehe Figur 3): Die Suche beginnt an einem der vier direkt angrenzenden Gitterpunkte (oberer, unterer, linker, rechter Gitterpunkt). Der erste dieser Punkte wird nach dem Zufallsprinzip ausgewählt. Das Molekül wird auf diesen Punkt gesetzt, wobei es keine Rolle spielt, ob der Punkt frei ist oder nicht. Wenn der Punkt vorher frei war, wird die Suche erfolgreich beendet. War der Punkt dagegen vorher besetzt, so wird die Suche mit den nächsten drei angrenzenden Gitterpunkten fortgesetzt. Wird kein freier Punkt gefunden, so werden die vier diagonalen Nachbarn (an den Ecken) untersucht. Ist auch dort kein freier Punkt vorhanden, so verbleibt das betreffende Molekül an dem Ort, an den es gesetzt wurde, d.h. auf dem ersten der angrenzenden Gitterpunkte. Für den Punkt, der sich ursprünglich an dieser Stelle befand, wird die gleiche Suche durchgeführt. Wenn nach einer vorbestimmten Anzahl n von Iterationen ein Molekül übrig bleibt, so wird dieses Molekül verworfen. Es wurde jedoch empirisch gefunden, daß schon mit n = 4 keine Moleküle zu verwerfen sind.

Es sei darauf hingewiesen, daß die Grenze des Gitters als besetzte Punkte angenommen wird. Wird ein Grenzpunkt erreicht, so wird die Suche fortgesetzt, bis sie sich wieder im Gitterbereich befindet.

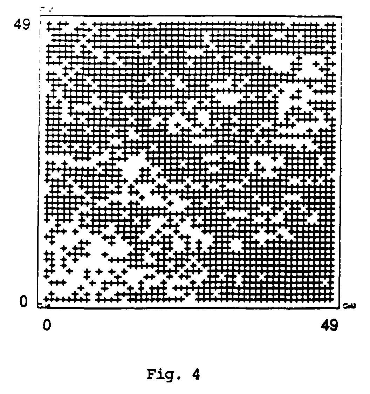

Als Ergebnis dieses Schritts ergibt sich eine Molekülverteilung über das Gitter, bei der kein Gitterpunkt von mehr als einem Molekül besetzt ist. Fig. 4 zeigt die resultierende Verteilung. Es sei außerdem darauf hingewiesen, daß bei dieser Entclusterungsmethode die Nachbarschaftsbeziehungen zwischen den Molekülen gewahrt bleiben.

Bis hierher ist das erfindungsgemäße Verfahren nicht speziell für einen bestimmten Rezeptor oder ein bestimmtes Target ausgelegt. Die folgenden Schritte sind jedoch für den Rezeptor oder das Target spezifisch. Es sei daran erinnert, daß die Kandidatenmoleküle mit der höchsten biologischen Aktivität hinsichtlich des Rezeptors oder Targets gefunden werden sollen. Die biologische Aktivität eines Moleküls kann auf vielen verschiedenen Wegen bestimmt werden, nämlich durch Berechnungen, Simulationen oder Versuche.

Da die Bestimmung der biologischen Aktivität aller Moleküle im Gitter zu zeitaufwendig wäre, muß eine Strategie zur Verringerung der Zahl der möglichen Kandidaten für die weitere Evaluierung angewandt werden. Dabei besteht die Schlüsselidee darin, daß benachbarte Moleküle auf dem Gitter aufgrund ihrer ähnlichen Eigenschaften (wie sie durch die Deskriptoren ausgedrückt werden) ähnliche Werte für die biologische Aktivität aufweisen. Es sei jedoch darauf hingewiesen, daß konkrete Werte für die biologische Aktivität nicht direkt aus den Deskriptoren bekannt sind. Diese Werte müssen nach den oben aufgeführten (sehr zeitaufwendigen) Methoden bestimmt werden.

Der geradlinigste Weg zur Suche der Moleküle mit den höchsten Werten für die biologische Aktivität über die Molekülverteilung hinweg bestünde in der Berechnung aller Werte des Molekülgitters. Eine derartige erschöpfende Herangehensweise wäre jedoch viel zu zeitaufwendig. Daher muß eine approximative Modellierung der Oberfläche durchgeführt werden.

Bei einer approximativen Modellierung der Oberfläche durch Bestimmung der Aktivitätswerte von Molekülen auf Gitterpunkten, die sich auf dem gesamten Gitter in regelmäßigem Abstand befinden, liefe man Gefahr, einige interessante Minimal- oder Maximalwerte zu übergehen.

Daher wird eine adaptive Suche durchgeführt, bei der interessante Bereiche des Gitters genauer evaluiert werden als andere Gitterbereiche.

Die Verteilung der biologischen Aktivität über das Molekülgitter wird als 3-D-Oberfläche modelliert, die die biologische Aktivität der Moleküle über das Gitter darstellt. Das Problem der Auffindung von geeigneten Molekülen wird in das Problem der Auffindung der Gitterpunkte (d.h. der Moleküle) mit den höchsten Werten für die biologische Aktivität transformiert.

Für die Approximation der Oberfläche der biologischen Aktivität hat sich die Methode der Delauney-Triangulierung als am besten geeignet erwiesen. Hierbei erfolgt die Approximation einer Oberfläche durch iterative Zerlegung in Dreiecke, wobei die Zahl der Iterationen in stärker geformten Bereichen, d.h. Bereichen mit größeren Aktivitätswert-Differenzen, erhöht wird.

Zunächst werden die Aktivitätswerte für die vier Eckpunkte des Gitters berechnet. Auf der Basis dieser vier Punkte wird die Oberfläche durch zwei Dreiecke approximiert (d.h. zerlegt). Für jedes der erhaltenen Dreiecke wird ein Qualitätskriterium berechnet, das die Qualität der Approximation der Oberfläche durch das betreffende Dreieck definiert. Es hat sich herausgestellt, daß als Qualitätskriterium der folgende Ausdruck geeignet ist:

$$DF(i) = \frac{\sum_{j \in nb_i} \left\| \nabla_i - \nabla_j \right\| \cdot \sum_{j \in nb_i} \left\| f(j) \right\|}{\# nb_i} \lambda(i) \cdot \kappa$$

Das Qualitätskriterium $DF(i)$ ist proportional zur Summe aller Gradientenwerte $\nabla_i$, $\nabla_j$ an den Eckpunkten $i$, $j$ und zum Mittelwert der Funktionswerte $f(j)$ über die Zahl der Eckpunkte $\# nb_i$ des jeweiligen Dreiecks $i$. Außerdem ist das Qualitätskriterium umgekehrt proportional zur Oberfläche $\lambda(i)$ dieses Dreiecks. Der Faktor $\chi$ ist ein Skalierfaktor, der je nach den Anforderungen für die Darstellung der Oberfläche bestimmt wird. Somit führt die Verwendung dieses Qualitätskriteriums zu einer besseren Zerlegung von Bereichen, die Moleküle mit großen Aktivitätsdifferenzen enthalten. Da die Oberfläche der Dreiecke berücksichtigt wird, werden große Dreiecke selbst dann behandelt, wenn sie keine Moleküle mit besonders großen Aktivitätsdifferenzen enthalten.

Als weiterer Gitterpunkt, dessen Aktivität berechnet wird, wird der Schwerpunkt des Dreiecks mit dem schlechtesten Qualitätsfaktor (entsprechend dem Gitterbereich unter der Oberfläche mit den größen Aktivitätsänderungen) gewählt. Auf der Basis dieses berechneten Punkts und aller zuvor berechneten Punkte wird eine neue Zerlegung der gesamten Gitteroberfläche in Dreiecke durchgeführt. Wiederum wird für jedes Dreieck der jeweilige Qualitätsfaktor berechnet, um einen weiteren Berechnungspunkt für die Verfeinerung der Zerlegung auszuwählen.

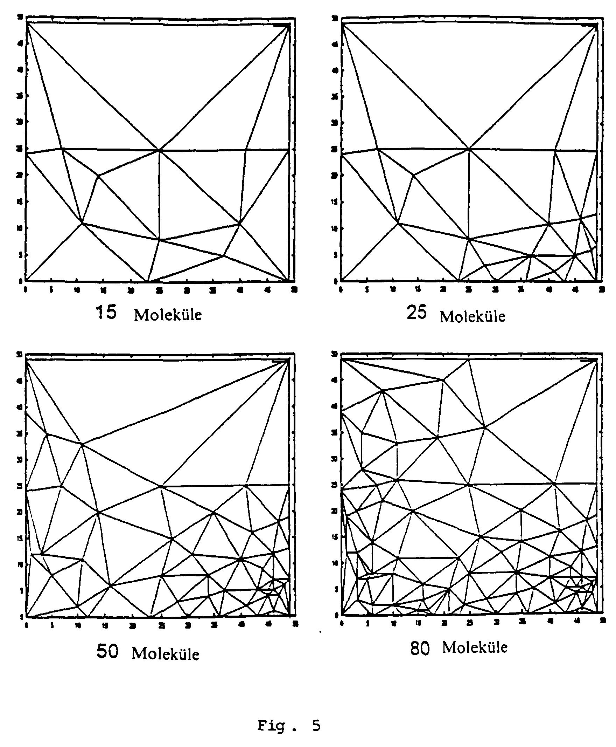

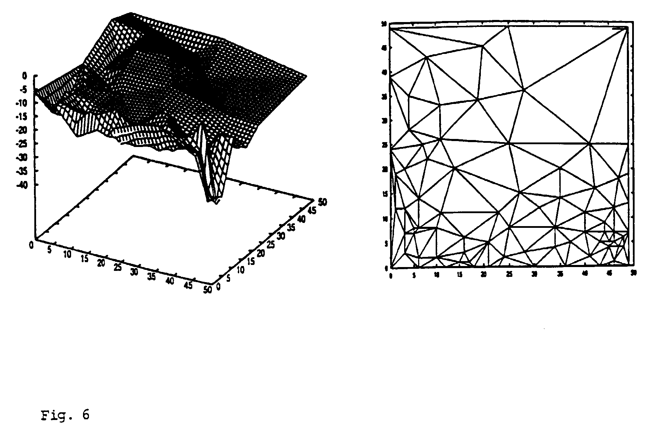

Dieser Schritt wird iterativ durchgeführt. Fig. 5 zeigt die jeweilige Zerlegung für vier Iterationsschritte, nämlich 15, 25, 50 und 80 berechnete Molekülgitterpunkte. Die Iteration wird beendet, wenn die Zerlegung genau genug geworden ist, d.h. wenn ein vorbestimmtes Stopkriterium, z.B. bei Berechnung von 5% aller Gitterpunkte, erreicht ist oder die allgemeine Fehlerfunktion (Summe aller Qualitätsfaktoren) einen vorbestimmten Schwellenwert erreicht. Die Gitteroberfläche kann dann als bezüglich der interessanten Bereiche des Gitters, d.h. der Bereiche mit Gitterpunkten mit den höchsten Werten für die biologische Aktivität, als optimal approximiert erachtet werden, wie in Fig. 6 zu sehen ist.

Im letzten Schritt werden dann alle Moleküle innerhalb eines vorbestimmten Radius um die Gitterpunkte mit der höchsten biologischen Aktivität explizit evaluiert.

Bei allen diesen Punkten handelt es sich um die resultierenden, als Kandidaten für die weitere pharmazeutische Forschung identifizierten Moleküle.

Durch Anwendung dieses Verfahrens können im Vergleich zu einer erschöpfenden Evaluierung mindestens 94% Rechenzeit eingespart werden. Außerdem werden infolge des Vorsortierungsschritts praktisch in jedem Fall die optimalen Kandidatenmoleküle gefunden.

Die identifizierten Moleküle können in geeigneten biologischen Assays geprüft werden, beispielsweise gemäß R. Bolger, "High-throughput screening: new frontiers for the 21$^{st}$ century", veröffentlicht in DDT, Band 4, Nr. 6, S. 251-253, Juni 1999, oder J. S. Major, "Challenges of high throughput screening against cell surface receptors", J. of Receptor and Signal Transduction Research, 15(1-4), S. 595-607. 1995).

Das erfindungsgemäße Verfahren wurde anhand eines Datensatzes von 40.000 Molekülen von dem World Drug Index (WDI) evaluiert. Einige Modifizierungen im Vergleich zu dem Basisverfahren wurden durchgeführt, um die Verarbeitung zu beschleunigen.

Für alle Moleküle wurde der molekulare Deskriptor berechnet. Der Sortierschritt fand auf einer rechtwinkligen selbstorganisierenden Karte (SOM) von 250 x 250 Neuronen (d.h. auf 62.500 Gitterpunkten) statt. Anstelle des willkürlichen Setzens der Moleküle auf das Gitter (Fig. 1) wurden die Gewichtungsvektoren der 62.500 Neuronen der selbstorganisierenden Karte initialisiert gemäß den ersten Eigenvektoren der vollständigen Moleküldeskriptormatrix, d.h. der 40.000 x 6-Matrix. Daher konnte der oben beschriebene Schritt des Entclusterns übersprungen werden.

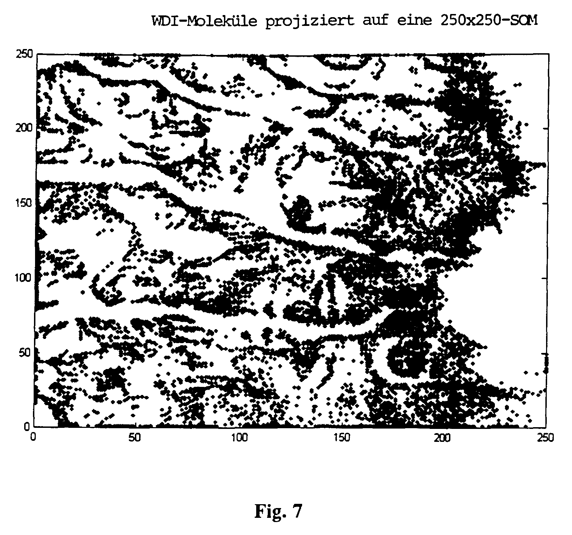

Das Lernen des SOM wurde mit einer Konvergenz von 0,2 und einer Verschiebung von 15 durchgeführt. Eine Gauß'sche Nachbarschaftsfunktion wurde verwendet. Das Lernen wurde in 5 Phasen durchgeführt, während die Verschiebung schrittweise auf 1 proportional zu der verstrichenen Lernzeit herabgesetzt wurde. Fig. 7 zeigt den sortierten Datensatz auf der Karte.

Dann wurde die Verteilung der biologischen Aktivität modelliert. Vor den Triangulierungsschritten wurde eine konvexe Hülle um alle Moleküle auf dem Gitter berechnet, wobei 8 bis 12 Punkte auf dem Gitter verwendet wurden. Die sich ergebenden Punkte wurden als Teil der Ausgangsdatenpunkte verwendet. Die anderen verwendeten Punkte sind der Mittelpunkt und die Mittelpunkte der vier Quadranten.

Bei diesen Punkten wurde die biologische Aktivität der darunter liegenden Moleküle bestimmt, und die erste Triangulation wurde berechnet. Die Docking-Berechnungen wurden sowohl für Thrombin- als auch für Dihydrofolatreduktase (DHFR)-Rezeptoren berechnet. Für jedes der sich ergebenden Dreiecke wurde das Qualitätskriterium $DF(i)$ berechnet. Die Zahl der Iterationen für die Triangulierung war 2000.

Als letzter Schritt wurden Kandidatenmoleküle mit einer Bindungsenergie unterhalb von -30 kJ/mol ausgewählt, und die biologische Aktivität aller Moleküle, die in dem ersten und zweiten Nachbarschaftskreis um diese Punkte herum liegen, wurde bestimmt. Die Gesamtmenge gefundener Moleküle mit einer Aktivität unterhalb von -30 kJ/mol wurde an diesem Punkt berechnet und verwendet als ein Maß für die Leistungsfähigkeit des Algorithmus.

Der vollständige Algorithmus wurde auf die beiden Rezeptoren Thrombin und DHFR angewandt. Wie zuvor dargestellt, mußte die SOM lediglich einmal berechnet werden. Für beide Rezeptoren wurde die Berechnung nach 2000 Iterationen beendet, d.h. 5% der Moleküle wurden berechnet.

Das gesamte Verfahren mit der anschließenden Verfeinerung benötigte etwa 4000 Minuten für das Docking (circa 2 Minuten pro Molekül) und 12 Stunden für die SOM-Berechnung, die nur einmal durchgeführt werden mußte. Dies führt zu einer Beschleunigung um den Faktor 8 für eine Untersuchung und zu einer Beschleunigung von 20 für alle weiteren Durchläufe für weitere Targets basierend auf derselben SOM.

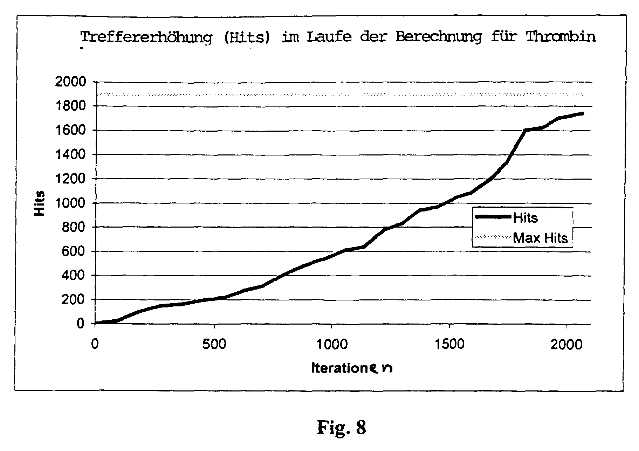

Fig. 8 zeigt die Menge aktiver Moleküle (Treffer oder Hits), die während der Iterationen für den Thrombin-Rezeptor gefunden wurden. Der Algorithmus war nach 2000 Interationen zu Ende. Die horizontale Graulinie zeigt die Gesamtmenge von Treffern, die in dem WDI für diesen speziellen Receptor vorhanden sind.

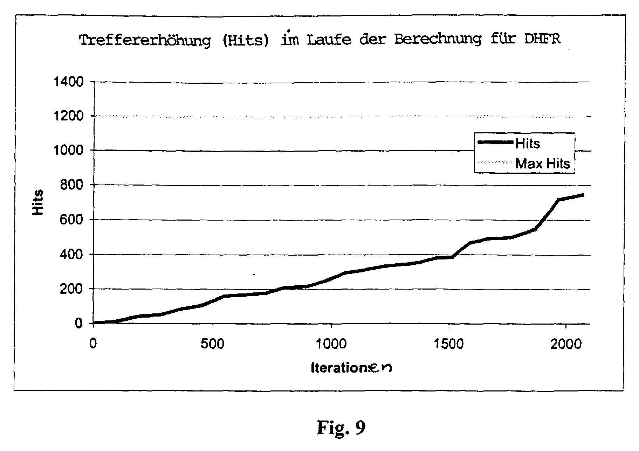

Fig. 9 zeigt die Menge von Treffern während der 2000 Iterationen für den DHFR-Rezeptor. Die horizontale Graulinie zeigt die totale Menge von Treffern, die in dem WDI für diesen speziellen Rezeptor vorhanden sind.

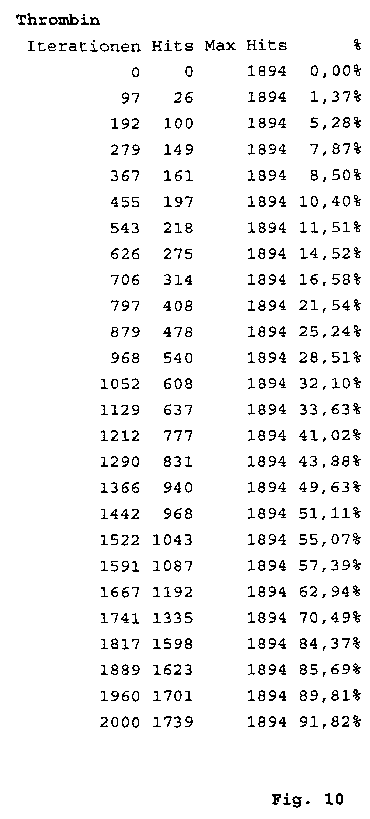

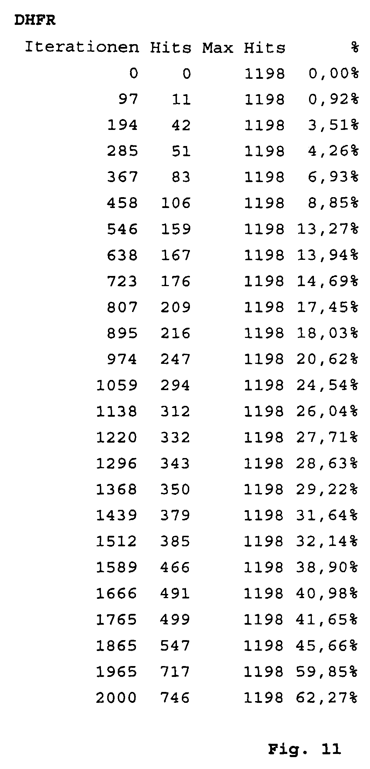

Fig. 10 und 11 zeigen den Prozentsatz von Treffern, bezogen auf die Gesamtmenge von Treffern als Funktion der Zahl von Iterationen für Thrombin bzw. DHFR.

## Patentansprüche

1. Verfahren zur Identifizierung von Kandidatenmolekülen mit erwarteter biologischer Aktivität, bei dem man:

    a) einen Satz aus verschiedenen Molekülen erzeugt;
    b) jedem der Moleküle des Satzes einen Deskriptor zuordnet, der eine vorbestimmte Zahl von molekularen Eigenschaften repräsentiert;
    c) den Molekülsatz auf Punkten eines zweidimensionalen Gitters hinsichtlich einer vorbestimmten Similaritätsbeziehung der jeweils zugeordneten Deskriptoren so kartiert, daß der Gitterabstand zwischen Gitterpunkten zweier Moleküle ein Maß für die Similarität der beiden Moleküldeskriptoren darstellt;
    d) über dem Molekülgitter eine dreidimensionale Oberfläche erstellt, die die Verteilung der biologischen Aktivität der Moleküle auf dem Gitter gemäß einem vorbestimmten Qualitätskriterium approximativ darstellt; und
    e) aus der dreidimensionalen Oberfläche Kandidatenmoleküle auswählt, die ein vorbestimmtes Kriterium hinsichtlich ihrer biologischen Aktivität erfüllen.

2. Verfahren nach Anspruch 1, bei dem man in Schritt d) die dreidimensionale Oberfläche dadurch erstellt, daß man:

    da) als Anfangsbereich für die Approximation das gesamte zweidimensionale Gitter nimmt;
    db) auf vorbestimmten Gitterpunkten dieses Bereichs Moleküle auswählt und deren jeweilige Werte für die biologische Aktivität berechnet;
    dc) die Oberfläche unter Verwendung der vorher bestimmten Werte für die biologische Aktivität der Moleküle auf den vorbestimmten Gitterpunkten über diesen Bereich approximiert und
    dd) bestimmt, ob die approximierte Oberfläche ein vorbestimmtes Qualitätskriterium erfüllt; wenn dies der Fall ist, geht man zu Schritt e); wenn dies nicht der Fall ist, verfeinert man die Approximation der Oberfläche durch Auswahl von Molekülen weiteren Gitterpunkten, Berechnung ihrer jeweiligen Werte für die biologische Aktivität und Wiederholung von Schritt dc) und diesem Schritt dd).

3. Verfahren nach Anspruch 1, bei dem man in Schritt d) die dreidimensionale Oberfläche durch eine Approximation von Dreiecken nach der Methode der Delauney-Triangulierung erstellt.

4. Verfahren nach Anspruch 1, bei dem die gewählten Kandidatenmoleküle für die chemische Synthese geeignet sind.

5. Verfahren nach Anspruch 1, bei dem es sich bei den von den Deskriptoren dargestellten molekularen Eigenschaften um mindestens zwei der folgenden Eigenschaften handelt:

    - Molekulargewicht,
    - Zahl der drehbaren Bindungen,
    - Zahl der hydrophoben Gruppen,
    - Zahl der hydrophilen Gruppen,
    - Zahl der sauren Gruppen,
    - Zahl der basischen Gruppen,
    - Zahl der neutralen Gruppen,
    - Zahl der Zwittergruppen,
    - Zahl der Schweratome,
    - Zahl der H-Bindungs-Donatoren,
    - Zahl der H-Bindungs-Akzeptoren,
    - Zahl der 1,2-Dipole,
    - Zahl der 1,3-Dipole,
    - Zahl der 1,4-Dipole.

6. Verfahren nach Anspruch 1, bei dem es sich bei den von den Deskriptoren dargestellten molekularen Eigenschaften um:

- Molekulargewicht,
- Zahl der drehbaren Bindungen,
- Zahl der hydrophoben Gruppen,
- Zahl der Schweratome,
- Zahl der H-Bindungs-Donatoren,
- Zahl der H-Bindungs-Akzeptoren

handelt.

7. Verfahren nach Anspruch 1, bei dem es sich bei den von den Deskriptoren dargestellten molekularen Eigenschaften um mindestens zwei der folgenden Eigenschaften handelt:

- Molekulargewicht,
- Zahl der drehbaren Bindungen,
- Zahl der hydrophoben Gruppen,
- Zahl der Schweratome,
- Zahl der H-Bindungs-Donatoren,
- Zahl der H-Bindungs-Akzeptoren.

8. Verfahren nach Anspruch 1, bei dem man die Molekülkartierung mit selbstorganisierenden Karten in einem neuronalen Netz oder mit statistischen Methoden wie linearer Vektorquantisierung durchführt.

9. Verfahren nach einem der vorhergehenden Ansprüche, bei dem man die Similaritätsbeziehung durch ein metrisches Maß der von den Deskriptoren dargestellten molekularen Eigenschaften definiert.

10. Verfahren nach Anspruch 1, bei dem man zwischen Schritt c) und d) die Moleküle auf dem Gitter derart neu kartiert, daß kein Gitterpunkt von mehr als einem Molekül besetzt ist, wobei die Similaritätsbeziehung zwischen zwei Punkten jeweils gewahrt wird.

11. Verfahren nach Anspruch 1, bei dem man die dreidimensionale Oberfläche durch rechnerische oder experimentelle Identifizierung der biologischen Aktivität erstellt.

12. Verfahren nach Anspruch 1, bei dem es sich bei dem vorbestimmten Kriterium für die biologische Aktivität um den Mindestwert der biologischen Aktivität handelt.

13. Verfahren nach Anspruch 1, bei dem der Molekülsatz in einer computergestützten Datenbank gespeichert wird.

14. Verfahren nach Anspruch 1, bei dem es sich bei den vorbestimmten Positionen des Schritts db) um die Eckpunkte des jeweiligen Bereichs handelt.

15. Verfahren nach Anspruch 3, bei dem das Qualitätskriterium durch

$$DF(i) = \frac{\sum_{j \in nb_i} \left\| \nabla_i - \nabla_j \right\| \cdot \sum_{j \in nb_i} \left\| f(j) \right\|}{\# nb_i} \lambda(i) \cdot \kappa$$

wobei:

$i$:       das jeweilige Dreieck,
$j$:       die Eckpunkte des Dreiecks $i$,
$f(j)$:     den Wert für die biologische Aktivität des Punkts $j$,
$\nabla_i, \nabla_j$:   den Wert einer auf den Punkt $i$, $j$ angewandten Gradientenoperation,
$\lambda(i)$:    die Oberfläche des Dreiecks $i$,
$\chi$:      ein Skalierfaktor und
$\# nb$:    die Zahl der Eckpunkte bedeutet,

definiert ist.

16. Verfahren nach Anspruch 2, bei dem das Stopkriterium durch die Summe über alle in einem Schritt berechneten Werte des Qualitätskriteriums definiert ist.

17. Verfahren nach Anspruch 2, bei dem das Stopkriterium durch eine vorbestimmte Zahl von berechneten Gitterpunkten definiert ist.

18. Verfahren nach Anspruch 1, bei dem man in Schritt e) auf dem Gitter um jedes ausgewählte Molekül die Werte für die biologische Aktivität einer vorbestimmten Zahl von benachbarten Molekülen berechnet.

19. Verfahren nach Anspruch 1, bei dem man die zugeordneten Punkte des 2-D-Gitters visualisiert.

20. Verfahren nach Anspruch 1, bei dem man die erhaltenen 3-D-Oberflächen visualisiert.

21. Computersystem mit Mitteln zur Durchführung des Verfahrens gemäß Anspruch 1.

22. Computersystem nach dem vorhergehenden Anspruch mit Mitteln zur Verbindung mit einer den Molekülsatz enthaltenden Datenbank.

23. Datenspeichermittel mit gespeichertem Programm zur Durchführung des Verfahrens gemäß Anspruch 1.

24. Datenspeichermittel mit gespeicherter Datenbank, die den Molekülsatz zur Verwendung bei dem Verfahren gemäß Anspruch 1 enthält.

25. Programm zur Speicherung einer Datenbank, die den Molekülsatz zur Verwendung bei dem Verfahren gemäß Anspruch 1 enthält.

26. Datenbank zur Verwendung bei dem Verfahren gemäß Anspruch 1.

27. Verfahren zur Herstellung von Molekülen nach dem Verfahren gemäß Anspruch 1.

28. Verfahren nach Anspruch 27, das außerdem auch noch einen Schritt f) umfaßt, bei dem man die gefundenen Kandidatenmoleküle in einem geeigneten biologischen Assay prüft.

```
6
269.80 2 5 4 1 3 BTB_03049
229.80 6 5 1 2 4 SPB_00387
259.85 4 3 2 1 1 EN_00236
179.05 4 2 0 0 2 NRB_00265
```

Fig. 1

Fig. 2

Fig. 3

Fig. 4

15 Moleküle

25 Moleküle

50 Moleküle

80 Moleküle

Fig. 5

Fig. 6

WDI-Moleküle projiziert auf eine 250x250-SOM

**Fig. 7**

Treffererhöhung (Hits) im Laufe der Berechnung für Thrombin

Fig. 8

Treffererhöhung (Hits) im Laufe der Berechnung für DHFR

Fig. 9

**Thrombin**

| Iterationen | Hits | Max Hits | % |
|---:|---:|---:|---:|
| 0 | 0 | 1894 | 0,00% |
| 97 | 26 | 1894 | 1,37% |
| 192 | 100 | 1894 | 5,28% |
| 279 | 149 | 1894 | 7,87% |
| 367 | 161 | 1894 | 8,50% |
| 455 | 197 | 1894 | 10,40% |
| 543 | 218 | 1894 | 11,51% |
| 626 | 275 | 1894 | 14,52% |
| 706 | 314 | 1894 | 16,58% |
| 797 | 408 | 1894 | 21,54% |
| 879 | 478 | 1894 | 25,24% |
| 968 | 540 | 1894 | 28,51% |
| 1052 | 608 | 1894 | 32,10% |
| 1129 | 637 | 1894 | 33,63% |
| 1212 | 777 | 1894 | 41,02% |
| 1290 | 831 | 1894 | 43,88% |
| 1366 | 940 | 1894 | 49,63% |
| 1442 | 968 | 1894 | 51,11% |
| 1522 | 1043 | 1894 | 55,07% |
| 1591 | 1087 | 1894 | 57,39% |
| 1667 | 1192 | 1894 | 62,94% |
| 1741 | 1335 | 1894 | 70,49% |
| 1817 | 1598 | 1894 | 84,37% |
| 1889 | 1623 | 1894 | 85,69% |
| 1960 | 1701 | 1894 | 89,81% |
| 2000 | 1739 | 1894 | 91,82% |

**Fig. 10**

**DHFR**

| Iterationen | Hits | Max Hits | % |
|---:|---:|---:|---:|
| 0 | 0 | 1198 | 0,00% |
| 97 | 11 | 1198 | 0,92% |
| 194 | 42 | 1198 | 3,51% |
| 285 | 51 | 1198 | 4,26% |
| 367 | 83 | 1198 | 6,93% |
| 458 | 106 | 1198 | 8,85% |
| 546 | 159 | 1198 | 13,27% |
| 638 | 167 | 1198 | 13,94% |
| 723 | 176 | 1198 | 14,69% |
| 807 | 209 | 1198 | 17,45% |
| 895 | 216 | 1198 | 18,03% |
| 974 | 247 | 1198 | 20,62% |
| 1059 | 294 | 1198 | 24,54% |
| 1138 | 312 | 1198 | 26,04% |
| 1220 | 332 | 1198 | 27,71% |
| 1296 | 343 | 1198 | 28,63% |
| 1368 | 350 | 1198 | 29,22% |
| 1439 | 379 | 1198 | 31,64% |
| 1512 | 385 | 1198 | 32,14% |
| 1589 | 466 | 1198 | 38,90% |
| 1666 | 491 | 1198 | 40,98% |
| 1765 | 499 | 1198 | 41,65% |
| 1865 | 547 | 1198 | 45,66% |
| 1965 | 717 | 1198 | 59,85% |
| 2000 | 746 | 1198 | 62,27% |

**Fig. 11**